(19) **Europäisches Patentamt European Patent Office Office européen des brevets**

(11) **EP 3 648 656 B1**

(12) **EUROPEAN PATENT SPECIFICATION**

(45) Date of publication and mention
of the grant of the patent:
**22.12.2021 Bulletin 2021/51**

(21) Application number: **18739807.8**

(22) Date of filing: **03.07.2018**

(51) Int Cl.:
***A61B 1/005*** (2006.01)

(86) International application number:
**PCT/EP2018/067992**

(87) International publication number:
**WO 2019/007981 (10.01.2019 Gazette 2019/02)**

(54) **CONTROL DEVICE FOR AN ELECTROSURGICAL INSTRUMENT**

STEUERUNGSVORRICHTUNG FÜR EIN ELEKTROCHIRURGISCHES INSTRUMENT

DISPOSITIF DE COMMANDE POUR UN INSTRUMENT ÉLECTROCHIRURGICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.07.2017 GB 201710701**

(43) Date of publication of application:
**13.05.2020 Bulletin 2020/20**

(73) Proprietor: **Creo Medical Limited
Chepstow, Monmouthshire NP16 5UH (GB)**

(72) Inventors:
• **HANCOCK, Christopher Paul
Bath Bath and North
East Somerset BA1 4LN (GB)**

• **TURNER, Louis
Chepstow Monmouthire NP16 5UH (GB)**

(74) Representative: **Mewburn Ellis LLP
Aurora Building
Counterslip
Bristol BS1 6BX (GB)**

(56) References cited:
**EP-A1- 2 361 581      WO-A1-2016/156542
GB-A- 2 545 465      US-A- 4 790 624
US-A- 5 556 370      US-A1- 2001 032 001
US-A1- 2017 056 105**

**Description**

FIELD OF THE INVENTION

The invention relates to a device for controlling an electrosurgical instrument that is configured to apply radiofrequency (RF) energy and/or microwave frequency energy to biological tissue. In particular, the present invention relates to a control or actuation mechanism for rotating a distal tip of the electrosurgical instrument. The actuation mechanism may enable the orientation of the instrument relative to a treatment site to be remotely controlled.

BACKGROUND TO THE INVENTION

Electrosurgical instruments are instruments that are used to deliver radio frequency and/or microwave frequency energy to biological tissue, for purposes such as cutting biological tissue or coagulating blood. Radio frequency and/or microwave frequency energy is supplied to the electrosurgical instrument using a transmission line, such as a coaxial cable, waveguide, microstrip line or the like.

It is known to use coaxial cables to deliver microwave and/or radio frequency energy along an instrument channel of a surgical scoping device to an electrosurgical instrument at the distal end of that channel. Such coaxial feed cables normally comprise a solid or flexible cylindrical inner conductor, a tubular layer of dielectric material around the inner conductor, and a tubular outer conductor around the dielectric material. The dielectric and/or outer conductor can be multi-layer structures.

An electrical connection is generally formed between the inner and outer conductors of the coaxial feed cable and corresponding conductor elements of an instrument tip by soldering a conductor such as a piece of wire or foil to the inner/outer conductor and to the corresponding conductor element. Radiofrequency energy and/or microwave frequency energy can thus be communicated from the coaxial feed cable to the instrument tip for delivery into the biological tissue.

Electrosurgical instruments have been used in conjunction with endoscopes, for example to cut or ablate a small portion of tissue in the gastrointestinal (GI) tract. In this context, the electrosurgical instrument is passed through an instrument channel of the endoscope, so that the instrument tip protrudes from the distal end of the endoscope where it can be brought into contact with the GI tract.

In some cases it may be desirable to rotate the instrument tip, in order to orient the instrument tip in a particular manner with respect to the treatment site. Rotation may be performed by turning the tip relative to the rest of the instrument, e.g. by application of a suitable rotating force at the distal end of the instrument. In some examples that can be enabled by providing a rotatable connection between the instrument tip and the coaxial feed cable which maintains the electrical connection between the instrument tip and coaxial feed cable when the instrument tip is rotated. WO 2016/059228 discloses an example of such a connection. A mechanical actuation mechanism may be provided to adjust the rotation of the instrument tip relative to the coaxial feed cable.

Other mechanisms for rotating the instrument tip are also known. In some electrosurgical instruments, the coaxial feed cable is contained within a flexible shaft, and the instrument tip protrudes from a distal end of the flexible shaft. The instrument tip is rigidly connected to the flexible shaft, such that when a user rotates the flexible shaft, the instrument tip also rotates. This enables the user to control the rotation of the instrument tip by rotating a proximal end of the flexible shaft. However, such an approach does not provide good control over the position of the instrument tip.

US4790624A discloses that a method and apparatus for spatially orienting a remote flexible member such as the tip of a borescope to point in a desired direction employs heating to its transition temperature a shape memory effect (SME) alloy actuating element coupled to the tip and cable. In one embodiment, an elongated helical spring fabricated from an SME alloy such as NI--TI or CU--ZN--AL is longitudinally disposed between the remote end of a borescope cable, and the borescope tip. An electrical resistance wire heater in contact with the spring and energized by an electrical power source controlled by an observer at the near end of the cable is used to heat a selected part of the spring to the transition temperature of the SME alloy of which it is fabricated, producing a controlled deflection of the borescope tip to point in a direction directed by the observer.

SUMMARY OF THE INVENTION

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes.

At its most general, the invention proposes the use of a shape memory effect (SME) material (e.g. a shape memory alloy, such as nitinol) in an actuator for effecting rotation of an electrosurgical instrument, and in particular for controlling rotation of an instrument tip at the distal end of an instrument channel in a surgical scoping device. For example, the actuator may be part of a control mechanism for rotating a tip of an electrosurgical instrument relative to a flexible shaft that encases components of the instrument as they are conveyed along an instrument channel of a surgical scoping device, such as an endoscope, bronchoscope, laparoscope, gastroscope, or the like.

The SME material in the actuator may be configured such that when it experiences a change in temperature, its shape alters in a manner that cause the instrument tip to rotate. By controlling the temperature change (e.g. through the delivery and/or removal of energy to the SME material), relative rotation of the instrument tip may be accurately controlled.

The change in temperature of the SME may be cause by delivered energy that to heat the material. Energy may be delivered directly, e.g. by running an electrical current through the material and causing it to heat up via the Joule effect. Alternatively the SME material may be heated using a heater which is in thermal contact with the SME material. The electrosurgical device can be configured to be fed through the instrument channel of a surgical scoping device (e.g. an endoscope), so that it can be used to carry out minimally invasive surgical procedures.

The electrosurgical instrument of the invention uses an electrical current rather than movable control wires to adjust the rotation of the instrument tip. This reduces the number of moving parts in the electrosurgical instrument, which may increase the reliability of the instrument as well as the accuracy with which the instrument tip can be rotated. In particular, the inventors have realised that it may be difficult to achieve smooth and accurate control using a movable control wire, as friction between the control wire and other parts of the instrument may cause it to move in a jerky, stick-slip manner. This effect may be especially pronounced when there are several bends in the instrument channel of the surgical scoping device (e.g. because the surgical scoping device is in the GI tract).

According to the invention, there is provided a control mechanism for rotating an instrument tip located at the distal end of an instrument channel in a surgical scoping device, the control mechanism comprising: an actuator formed from a shape memory effect material; and an energy delivery structure connected to the actuator to deliver energy to cause a change in temperature of the shape memory effect material, wherein the actuator is configured to exhibit a torque between a proximal end and a distal end thereof in response to the shape memory effect material reaching a threshold temperature. The invention thus makes use of a behaviour of the shape memory effect material to return to an original shape when its temperature exceeds a certain threshold. The actuator is shaped such that the return to the original shape involves a rotational or twisting motion that can be used to impart a torque between an instrument tip and its surroundings.

The shape memory effect material is a material that, when deformed, returns to its original (i.e. pre-deformed) shape when it is heated above a transition temperature. In order to cause rotation of the instrument tip when the shape memory effect material is heated, the shape of the shape memory effect material may be trained in an appropriate manner (e.g. by twisting it and heat-treating it) during its manufacture before installing it in the electrosurgical instrument. Then, when the actuator is heated, it deforms (e.g. it untwists) and causes the instrument tip to rotate.

In some cases, the shape memory effect material may exhibit a two-way memory effect. Materials exhibiting the two-way memory effect can be trained such that they can be repeatedly cycled between two predetermined shapes by thermally cycling them. The use of such a two-way memory effect material enables the rotation of the instrument tip to be adjusted in both clockwise and anti-clockwise directions by controlling the temperature of the material. Suitable shape memory effect materials include, but are not limited to, shape memory alloys (e.g. nickel-titanium (NiTi), iron-based and copper-based SME alloys), and shape memory effect polymers. In some preferred embodiments the actuator is made of NiTi, which is formed of 45% Ti and 55% Ni. An advantage of NiTi is that it can be used to produce large forces and torques, as well as large deformations when it is heated above its transition temperature (NiTi has a transition temperature of approximately 40°C).

In one example, the shape memory effect material may comprise a helical structure, which may provide an efficient physical configuration for applying a useful torque. The helical structure may be a wound wire or sheet or material. The shape memory effect material may be have an original configuration (above the threshold temperature) that is less tightly wound than a starting configuration. Thus, the shape memory effect material may be configured to unwind upon the temperature of the shape memory effect material reaching the threshold temperature. The threshold temperature may be selected to avoid accidental rotation, e.g. caused by body heat or heat arising through delivery of energy at the treatment site, etc. For example, the threshold temperature may be equal to or greater than 40°C.

In one example, the shape memory effect material may comprise a pair of cooperating helical structures. This structure can provide more torque, and is also less vulnerable to breakage.

The control mechanism may include a power source connected to the energy delivery structure. Any suitable type of energy can be used to affect the temperature of the actuator. For example, the energy delivery structure may comprise a conductive element (e.g. cable) for running an electrical current through the actuator. The energy delivery structure may provide a pair of conductive elements running from each end of the actuator in order to provide a complete current path. The power source in this example may be a DC current source or the like. The power source may be controllable to deliver a desired amount of energy to the actuator. The power source may deliver the energy continuously or in a pulsed manner.

The control mechanism may comprise a temperature sensor arranged to detect a temperature of the actuator. An output from the temperature sensor may be used as feedback to control operation of the power source.

In another example, the energy delivery structure may comprise a heater thermally connected to the actuator. The heater may be a resistive heater, e.g. connected to a DC source.

The control mechanism may include a coolant delivery structure arranged to extract thermal energy from the actuator. This can enable closer control over the temperature of the actuator, to give more accuracy to the position of the instrument tip. The coolant delivery structure may comprise a coolant circuit in thermal communication with the actuator, e.g. via a heat exchanger or the like. The coolant delivery structure may be selectively operable, i.e. to allow the cooling effect to be switched in only when needed. Alternatively, the cooling effect may be constantly present, with energy for heating the actuator being delivered in an adjustable manner to control the temperature of the shape memory effect material.

In another aspect, the invention provides an electrosurgical instrument for applying radiofrequency (RF) electromagnetic (EM) energy and/or microwave EM energy to biological tissue, the instrument comprising: a coaxial cable for conveying the RF EM energy and/or the microwave EM energy; an instrument tip connected at a distal end of the coaxial cable to receive the RF EM energy and/or the microwave EM energy and deliver it into biological tissue at a treatment site; an elongate shaft defining a lumen for conveying the coaxial cable, wherein the instrument tip protrudes from a distal end of the flexible shaft; and a control mechanism having any of the features defined above, wherein the proximal end of the actuator is attached to the shaft and the distal end of the actuator is attached to the instrument tip.

The shape memory effect material may form a sleeve (e.g. a helical sleeve) around a distal portion of the shaft. The shaft may comprise a hollow tube made of a suitable flexible material. The coaxial cable, together with any wiring and fluid conduits of the electrosurgical instrument may be contained within the shaft, and connected to the instrument tip at the distal end of the shaft. The shaft may be insertable through the entire length of an instrument (or working) channel of a surgical scoping device, such as an endoscope, bronchoscope, gastroscope, etc.

The coaxial cable may include an inner conductor, an outer conductor coaxial with the inner conductor, and a first dielectric material separating the inner and outer conductors. The coaxial feed cable may also include an outer protective sheath to protect and insulate the outer conductor. In some examples, the inner conductor may be formed of a hollow tube made of a conductive material, so that wires and/or fluid conduits may be fed through the inner conductor, e.g. to deliver fluid at the instrument tip.

The instrument tip may include one or more electrodes electrically connected to the inner and outer conductors of the coaxial feed cable so that it may deliver the RF EM and/or microwave EM energy to biological tissue. Many different instrument tip configurations are possible. For example, the instrument tip may include a bipolar emitting structure comprising a first electrode spatially separated from a second electrode by a second dielectric material. The first and second electrodes may be arranged to act respectively as active and return electrodes for conveying radio frequency energy, or as an antenna for radiating microwave frequency energy.

In some embodiments, the actuator may be fixed at a first end to the flexible shaft and fixed at a second end to the instrument tip. In this manner the instrument tip may be rotated by the actuator relative to the distal end of the flexible shaft when the actuator is heated. The actuator may be fixed to the flexible shaft and the instrument tip using any suitable fixing means, such as adhesive, mechanical fasteners and/or welding.

In one example, the instrument may comprise engagement features at the connection between the shaft and proximal end of the actuator. The engagement features may be configured to resist relative rotation between the shaft and actuator. The engagement features may for example comprise cooperating interengageable elements on the shaft and actuator.

Similarly, there may be engagement features (e.g. cooperating interengageable elements) at the connection between the instrument tip and distal end of the actuator configured to resist relative rotation between the instrument tip and actuator.

The shaft may itself be fixed at a proximal end of the electrosurgical instrument, in order to prevent the flexible shaft from rotating. This ensures that any torque generated by the actuator when the actuator is heated is transmitted to the instrument tip, rather than to the flexible shaft which could cause the flexible shaft to become twisted. Additionally, the shaft may be made of a material which resists twisting motions, to further ensure that any torque generated by the actuator is transmitted to the instrument tip.

The coaxial cable and any other structures attached to the instrument tip (e.g. fluid conduits, wires) may be allowed to rotate within the instrument shaft when the instrument tip rotates. This prevents a build-up of stress at the interface between the coaxial feed cable and the instrument tip, which could disrupt the connection between the coaxial feed cable and the instrument tip.

In another aspect, the invention provides an electrosurgical system comprising: a generator for generating radiofrequency (RF) electromagnetic (EM) energy and/or microwave EM energy; an electrosurgical instrument having any of the features defined above connected to the generator; and a surgical scoping device having a manoeuvrable instrument cord with a instrument channel extending therethrough, wherein the electrosurgical instrument is dimensioned to pass through the instrument channel.

Herein, the terms "proximal" and "distal" refer to the ends of a structure (e.g. electrosurgical instrument, coaxial feed cable, etc.) further from and closer to the treatment zone respectively. Thus, in use the proximal end of the structure is accessible by a user, whereas the distal end is closer to the treatment site, i.e. the patient.

The term "conductive" is used herein to mean electrically conductive, unless the context dictates otherwise.

The term "longitudinal" used below refers to the direction along the instrument channel parallel to the axis of the coaxial transmission line. The term "lateral" refers to a direction that is perpendicular to the longitudinal direction.

The term "inner" means radially closer to the centre (e.g. axis) of the instrument channel. The term "outer" means radially further from the centre (axis) of the instrument channel.

The term "electrosurgical" is used in relation an instrument, apparatus or tool which is used during surgery and which utilises microwave electromagnetic (EM) energy and/or radiofrequency EM energy. Herein, microwave EM energy may mean electromagnetic energy having a stable fixed frequency in the range 300 MHz to 100 GHz, preferably in the range 1 GHz to 60 GHz. Preferred spot frequencies for the microwave EM energy include 915 MHz, 2.45 GHz, 5.8 GHz, 14.5 GHz, 24 GHz. 5.8 GHz may be preferred. Herein, radiofrequency EM energy may mean electromagnetic energy having a stable fixed frequency in the range 10 kHz to 300 MHz.

## BRIEF DESCRIPTION OF THE DRAWINGS

Examples of the invention are discussed below with reference to the accompanying drawings, in which:

Fig. 1 is a schematic diagram of an electrosurgical system within which the invention may be used;
Fig. 2 is a schematic diagram of an electrosurgical instrument that is an embodiment of the invention;
Fig. 3 is a schematic diagram of an actuator which may be used in an electrosurgical instrument of the invention;
Fig. 4A is a schematic diagram of a distal end of the actuator of Fig. 3;
Fig. 4B is a schematic diagram of a proximal end of the actuator of Fig. 3; and
Fig. 5 is a schematic cross-sectional view of a flexible shaft of an electrosurgical instrument according to the invention.

## DETAILED DESCRIPTION; FURTHER OPTIONS AND PREFERENCES

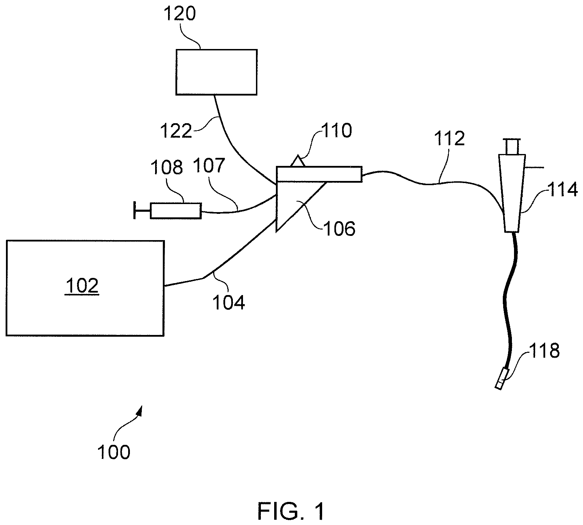

Fig. 1 is a schematic diagram of a complete electrosurgical system 100 that is an embodiment of the invention. The system is arranged to treat biological tissue (e.g. a tumour, lesion or fibroid) using radiofrequency (RF) energy and/or microwave frequency energy from an instrument tip. The system 100 comprises a generator 102 for controllably supplying RF energy and/or microwave energy. A suitable generator for this purpose is described in WO 2012/076844. The generator 102 is connected to an interface joint 106 by an interface cable 104. The interface joint 106 may also be connected to receive a fluid supply 107 from a fluid delivery device 108, such as a syringe. If needed, the interface joint 106 may house a needle movement mechanism that is operable by sliding a trigger 110. The function of the interface joint 106 is to combine the inputs from the generator 102, fluid delivery device 108 and needle movement mechanism, together with any other inputs which may be required into a single flexible shaft 112, which extends from the distal end of the interface joint 106.

The flexible shaft 112 is insertable through the entire length of an instrument (working) channel of an endoscope 114. The flexible shaft 112 has a instrument tip 118 that is shaped to pass through the instrument channel of the endoscope 114 and protrude (e.g. inside the patient) at the distal end of the endoscope's tube. The distal end assembly includes an active tip for delivering RF EM energy and/or microwave EM energy into biological tissue and optionally a retractable hypodermic needle for delivering fluid.

The structure of the instrument tip 118 may be arranged to have a maximum outer diameter suitable for passing through the working channel. Typically, the diameter of a working channel in a surgical scoping device such as an endoscope is less than 4.0 mm, e.g. any one of 2.8 mm, 3.2 mm, 3.7 mm 3.8mm. The length of the flexible shaft 112 can be equal to or greater than 1.2 m, e.g. 2 m or more. In other examples, the instrument tip 118 may be mounted at the distal end of the flexible shaft 112 after the shaft has been inserted through the working channel (and before the instrument cord is introduced into the patient). Alternatively, the flexible shaft 112 can be inserted into the working channel from the distal end before making its proximal connections. In these arrangements, the distal end assembly 118 can be permitted to have dimensions greater than the working channel of the surgical scoping device 114.

The system described above is one way of introducing the instrument into a patient. Other techniques are possible. For example, the instrument may also be inserted using a catheter.

The invention seeks to provide an instrument for applying RF energy and/or microwave frequency energy to biological tissue, where the instrument tip 118 can be rotated relative to the flexible shaft 112. The rotation of the instrument tip 118 is enabled by an actuator (not shown) which is formed of a shape memory effect (SME) material and which is located on the flexible shaft 112 near the instrument tip 118. As described in more detail below, the actuator is configured such that when it is heated it changes shape in a manner which causes the instrument tip 118 to rotate relative to the flexible shaft 112. The actuator may be heated either directly by passing an electrical current through it, or by using a heater which is thermally connected to the actuator. In order to heat the actuator in a controlled manner, the system 100 further includes a power source 120 which is connected to the interface joint 106 via cable 122. The power source 120 may be used to run a current through the actuator and/or to activate a heater which is thermally connected to the actuator. By controlling the current delivered by the power source 120, the change in temperature of the actuator may be controlled in order to accurately adjust the angle of rotation of the instrument tip 118 relative to the instrument shaft 112.

Although the power source 120 is shown as a separate component in Fig. 1, it may be understood that it could be incorporated into the generator (e.g. as a DC output therefrom) or as part of the scoping device 114, e.g. mounted or integrally formed thereon.

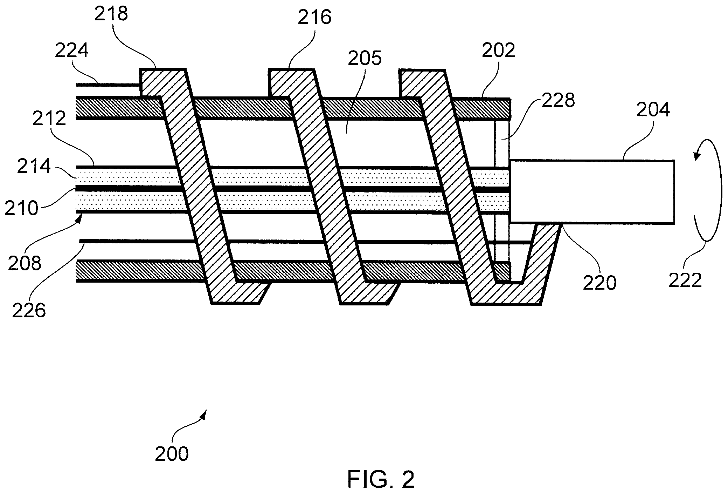

Fig. 2 shows a schematic diagram of an electrosurgical instrument 200 comprising a control mechanism that is an embodiment of the invention. The electrosurgical instrument 200 includes a shaft 202 (e.g. corresponding to shaft 112 discussed above), which is an elongate tubular structure that may be dimensioned to fit within the instrument channel of an surgical scoping device. The shaft 202 may be may of flexible material along all or part of its length so that is can adapt to the shape or configuration of the instrument channel as it is manoeuvred into place.

Protruding from a distal end of the shaft 202 is an instrument tip 204 which is configured to deliver radio frequency and/or microwave frequency energy to biological tissue. A suitable tip structure is disclosed in WO 2014/006369, for example, but the present invention need not be limited by any detail in tip structure.

The instrument tip 204 is electrically connected to a coaxial feed cable 208, which is arranged to convey RF EM energy and/or microwave EM energy (e.g. from generator 102) to the instrument tip 204. The coaxial feed cable 208 extends longitudinally within a lumen 205 formed by the flexible shaft 202. The coaxial feed cable 208 includes an inner conductor 210, which is separated from an outer conductor 212 by a first dielectric material 214. The coaxial feed cable 208 may be low loss for microwave frequency energy. A choke (not shown) may be provided on the coaxial feed cable 208 to inhibit back propagation of microwave energy reflected from the instrument tip 204 and therefore limit backward heating along the device.

Other elements (not shown) of the electrosurgical instrument 200 may also be contained within the lumen 205 of shaft 202, depending on the specific configuration of the electrosurgical instrument 200. For example, a fluid conduit (such as fluid supply 107), control wires (e.g. a needle movement mechanism operable by sliding trigger 110) and electrical wiring (e.g. for one or more sensors located near the instrument tip) may be contained within the flexible shaft 202. In some embodiments, the flexible shaft 202 may comprise a multi-lumen structure, where different elements of the electrosurgical instrument 200 are contained within respective lumens of the multi-lumen structure. The inner conductor 210 itself may be hollow, so that it may be used as a transport channel to convey instrument or other material (e.g. fluid, such as saline) to and from the instrument tip 204.

The electrosurgical instrument 200 further includes an actuator 216 made of a SME material (e.g. nitinol, NiTi) located at a distal end of the shaft 202. In this example, the actuator 216 is secured at a first end 218 to the flexible shaft 202, and at a second end 220 to the instrument tip 204. The actuator 218 may be fixed at its ends using any suitable means, such as adhesive, mechanical fasteners or welding. In the example shown in Fig. 2, the actuator 216 is a wire made of SME material which is coiled around a distal portion of the flexible shaft 202, i.e. the flexible shaft 202 passes through the coils of the actuator 216. For illustration purposes, the flexible shaft 202 and coaxial feed cable 208 have been represented as a cross-section, whilst the actuator 216 is illustrated wrapped around the outside of the flexible shaft 202. In practice, the inside of the flexible shaft 202 would not be visible. Although the actuator is wrapped around the outside of the shaft 202 in this embodiment, in other examples it may be mounted within the shaft 202, e.g. secured at the first end to an inner surface of the shaft 202.

The distal portion may extend for a distance equal to or less than 20 cm (preferably 10 cm) back from the distal end of the shaft 202. The actuator may thus be located in a portion of the shaft that is not subjected to significant bending within the instrument channel of the surgical scoping device.

The actuator is arranged such that when its temperature increases, it deforms in a manner which causes the instrument tip 204 to rotate relative to the flexible shaft, as indicated by arrow 222. In the example of Fig. 2, the coil of the actuator 216 is configured to unwind when its temperature increases. As the actuator 216 is held fixed relative to the flexible shaft 202 at its first end 218, the unwinding of the coil causes the instrument tip 204 to rotate relative to the flexible shaft 202. To achieve this effect, the actuator is initially formed in a first "unwound" configuration at a temperature above its transition temperature. After cooling to a temperature below the transition temperature, the actuator is deformed into a second "wound" configuration in which it can be mounted to the instrument tip and shaft. As the temperature of the actuator rises to the transition temperature, it will deform back to the first configuration, thereby rotating the instrument tip.

The flexible shaft 202 may be held fixed at a proximal end of the electrosurgical instrument (e.g. at the joint interface 106), in order to prevent it from rotating. This ensures that deformation of the actuator does not cause any unwanted rotation or twisting of the flexible shaft 202. Additionally, the flexible shaft 202 may include a structure which inhibits or prevents it from twisting along its length. Such a structure may ensure that deformation of the actuator 216 acts preferentially to rotate the instrument tip relative to the shaft because rotation of the tip presents least resistance to the torque imparted by the actuator.

The flexible shaft 202 may be formed of a braided tube having a braided wire (e.g. stainless steel) sleeve mounted between a radially inner polymer layer and a radially outer polymer layer. In order to avoid the braided wire from interfering with the delivery of radio frequency and/or microwave frequency energy to the instrument tip 204, a distal portion of the flexible shaft 202 (e.g. the distal portion around which the actuator is wound) adjacent the instrument tip 204 may be made of just the polymer layers, i.e. without an internal braid.

To cause a temperature change in the actuator 216, a current may be run through it. In the example shown in Fig. 2, a first wire 224 is connected to the first end 218 of the actuator 216, and a second wire 226 is connected to the second end 220 of the actuator 216. A power source (e.g. power source 120) can be connected to wires 224 and 226 to run a DC current through the actuator 216, which may cause the actuator 216 to heat up via the Joule effect. In this example, the first wire 224 runs along an outer surface of the shaft 202, whilst the second wire 226 is disposed inside the lumen 205. Other wiring configurations are also possible. For example both wires may run through the inside of the flexible shaft 202 and exit the flexible shaft 202 at its distal end to connect to the ends of the actuator 216.

The temperature of the actuator 216 may be monitored using a temperature sensor (not shown) located on or near the actuator 216. The actuator 216 may be electrically insulated, e.g. by a suitable insulating coating, such as a parylene or the like.

Alternatively, a heater (not shown) may be used to increase the temperature of the actuator 216. The heater may be mounted directly on the actuator 216, or it may be mounted near the actuator 216, with a thermal link being provided between the heater and the actuator. The heater may be a resistive chip which produces heat when a current is run through it. An advantage of using a heater may be that a lower current is required to produce a desired change in temperature of the actuator 216 compared to running a current directly through the actuator 216 (e.g. because the SME material may have a low electrical resistivity).

The coaxial feed cable 208 and any other components which are connected to the instrument tip may be free to rotate inside the flexible shaft 202, in order to facilitate rotation of the instrument tip 204. In cases where the coaxial feed cable 208 is fixed at a proximal end of the electrosurgical instrument 200 in a manner which prevents it from rotating, the coaxial feed cable 208 may be configured so that it can become twisted when the instrument tip 204 rotates. A fluid-tight seal 228 may be provided at the distal end of the flexible shaft 202, in order to prevent fluid from entering or exiting the flexible shaft 202. The fluid-tight seal 228 may have passage through which the instrument tip 204 protrudes, and which allows rotation of the instrument tip 204 relative to the flexible shaft 202. The passage may be shaped so as to form a fluid-tight seal with coaxial feed cable 208 and/or the instrument tip 204. Other passages may be provided in the seal 228, e.g. for wire 226. The seal 228 may also serve to keep the position of the instrument tip 204 fixed relative to the flexible shaft 202 when the instrument tip 204 is rotated, e.g. by preventing longitudinal and/or lateral motion of the instrument tip 204 relative to the flexible shaft 202.

In other embodiments, it may be desirable to not cause rotation and/or twisting of the coaxial feed cable 208 when the instrument tip 204 is rotated. This may be enabled by providing a flexible and/or rotatable connection between the coaxial feed cable 208 and the instrument tip 204. The rotatable connection may be arrange to maintain an electrical connection between the coaxial feed cable 208 and the instrument tip whilst the instrument tip 204 is rotated.

The actuator 216 may be configured to deform when it reaches a predetermined transition temperature. For example, an actuator made of NiTi may be configured to deform at a transition temperature around 40°C. By controlling the temperature of the actuator 216, the rotation of the instrument tip 204 relative to the flexible shaft 202 may be adjusted. For example, the actuator 216 may be heated by applying current pulses (either directly to the actuator or to the heater), so that the temperature of the actuator can be increased in a controlled manner. Alternatively, a continuous current may be applied. By monitoring the temperature of the actuator 216 using a temperature sensor, current may be applied to heat the actuator 216 to the transition temperature, at which the actuator begins to deform and causes the instrument tip 204 to rotate relative to the flexible shaft 202. To stop rotation of the instrument tip 204, the temperature of the actuator 216 may be allowed to drop below the transition temperature (e.g. by stopping the application of current). The actuator may be configured so that it can rotate the instrument tip through more than one full rotation as it returns to the first "unwound" configuration.

The SME material of the actuator may exhibit a one-way memory effect. The actuator may thus be a single use item, or may require manually resetting to the second "wound" configuration for subsequent use.

However, in some embodiments, the actuator may be formed of a "two-way" SME material. Materials exhibiting the "two-way" SME effect are trained such that they can be repeatedly cycled between two predetermined shapes by thermally cycling them. Thus, when the actuator 216 is made using such a material, heating up of the actuator 216 may cause rotation of the instrument tip 204 in one direction, whilst allowing the actuator 216 to cool down will cause rotation of the instrument tip 204 in the opposite direction.

In some cases, it may also be desirable to provide the instrument 200 with a mechanism for actively cooling the actuator 216 in use, in order to improve the accuracy of temperature control of the actuator 216. For example, the instrument 200 may include a cooling circuit (not shown), where a coolant (e.g. water, saline or the like) can be selectively applied to the actuator. In one example, the actuator may be in thermal communication with a heat exchanger through which the coolant can be selectively circulated. Alternatively, the coolant may continuously circulate in contact with the heat exchanger, whereby a balance between cooling power provided by the cooling circuit and heating power provided by the applied current may be adjustable in order to accurately control the temperature of the actuator. Such adjustability may be performed automatically, e.g. using PID control or the like.

In other examples, the cooling mechanism may include a nozzle arranged to spray a coolant directly onto the actuator in order to reduce its temperature.

Different shapes and configuration of the actuator 216 are possible. For example, the actuator may be a sleeve made of SME material in which a distal portion of the flexible shaft is contained, rather than a wire as shown in Fig. 2. Alternatively, the actuator may be formed of two or more cooperating coils made of SME material. In this manner, the torque load on each individual coil may be reduced. This may serve to increase the total amount of torque which can be applied by the actuator to the instrument tip, as well as reduce the risk of breakage of one of the coils.

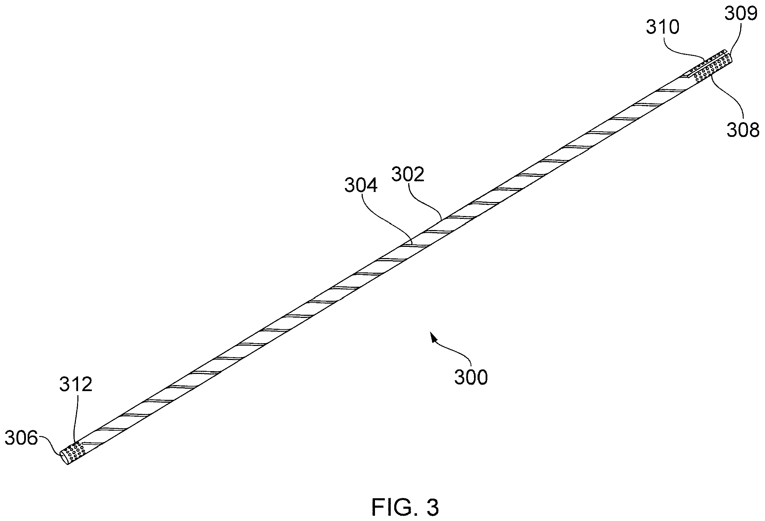

Fig. 3 shows a schematic diagram of an actuator 300 which may be used in an electrosurgical instrument according to the invention. The actuator 300 is formed of two cooperating helices (first helix 302 and second helix 304) made of a SME material (preferably NiTi). The actuator 300 may be used to rotate an instrument tip through 540 degrees (i.e. one and a half turns), due to the unwinding of the helices when the actuator 300 is raised above its transition temperature.

The two helices 302 and 304 are formed of wire having an approximately rectangular cross-section. The two helices 302 and 304 are joined at a distal end 306 of the actuator 300, such that the two helices are electrically connected at the distal end 306. The first helix 302 has a first electrical terminal 308 at a proximal end 309 of the actuator 300, and the second helix 304 has a second electrical terminal 310 at the proximal end 309. Thus, the first and second helices 302 and 304 form a continuous current path, and a current can be sourced through actuator 300 to heat it using the first electrical terminal 308 and the second electrical terminal 310. The actuator 300 may be integrally formed from a single piece of material.

The cooperating helices 302 and 304 form a sleeve through which the flexible shaft of an electrosurgical instrument may be passed. A connector 312 is formed at the distal end of the actuator 300 for fixing the actuator 300 to an instrument tip. The connector 312 includes a series of ultrasonic weld features, which may be used to ultrasonically weld an instrument tip to the connector 312. In the example shown in Fig. 3, the ultrasonic weld features are a series of holes which are arranged to receive complementary ultrasonic weld features (e.g. lugs which fit in the holes) on the instrument tip, in order to provide a strong connection between the actuator 300 and the instrument tip. Similar ultrasonic weld features are provided on the helices 302 and 304 at the proximal end 309 of the actuator, which enable the actuator 300 to be welded at its proximal end 309 to the flexible shaft via complementary ultrasonic weld features on the flexible shaft. The use of complementary ultrasonic weld features may enable torque to be efficiently transmitted from the actuator 300 to the instrument tip, and may prevent the actuator 300 from slipping relative to the flexible shaft and/or the instrument tip. Advantageously, the ultrasonic weld features on the actuator 300 may also be used to hold the actuator in pace when heat-treating the actuator as part of its manufacture process.

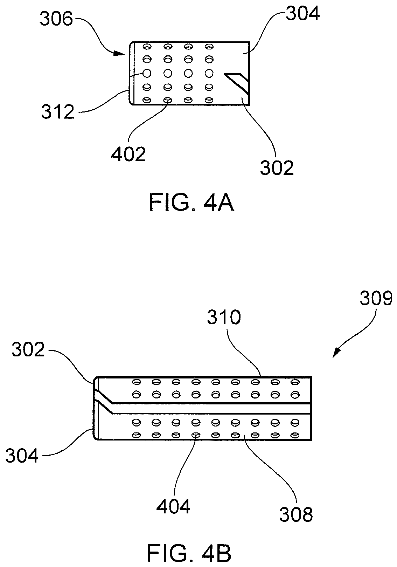

Figs. 4A and 4B show more detailed views of distal end 306 and proximal end 309 of the actuator 300, respectively. As can be seen in Fig. 4A, the two helices 302 and 304 are integrally formed with the connector 312, which mechanically and electrically connects them. The connector 312 includes a series of holes 402 which serve as ultrasonic weld features as described above. As show in Fig. 4B, the electrical terminal 308 and 310 at the proximal end 309 of the actuator have holes 404 formed therein which may be used as ultrasonic weld features as described above. The helices 302 and 304 may start at approximately 10 mm from the proximal edges of the electrical terminals 310 and 308, respectively.

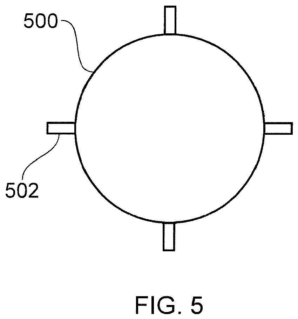

Fig. 5 shows a schematic cross-section of a flexible shaft 500 of an electrosurgical instrument according to the invention. The coaxial feed cable and any other components which may be contained in the flexible shaft 500 are not illustrated in Fig. 5. Multiple fixing lugs 502 are disposed on an outer surface of the flexible shaft 500. The fixing lugs 502 are configured to engage with complementary engagement features (e.g. holes 404) on the proximal end of the actuator (not shown), so that the actuator and the flexible shaft are rotationally locked relative to one another. The fixing lugs 502 may be formed of the same material as the flexible shaft 500, and may be integrally formed with the flexible shaft 500. The fixing lugs may be ultrasonic weld features, such that they may be used to ultrasonically weld the flexible shaft 500 to the actuator, in order to provide a strong bond between the two components. Similar fixing lugs may also be provided on the instrument tip, so that it can be rotationally fixed relative to the actuator, so that torque may efficiently be transmitted from the actuator to the instrument tip.

Example calculations will now be set out showing how mechanical requirements for an actuator for use in an electrosurgical instrument of the invention may be estimated.

In an experiment, the inventors found that to rotate an instrument cable (e.g. a coaxial feed cable for conveying radio frequency and/or microwave frequency energy) through 180 degrees when the cable is in 170 degree retroflex (i.e. the cable is bent by an angle of 170 degrees), it is necessary to provide a torque of approximately $56 \times 10^{-3}$ Nm. In order for the electrosurgical instrument to fit through an instrument channel of an endoscope, the inventors chose 3.10 mm as the maximum outer diameter for an actuator placed around a distal portion of a flexible shaft of the electrosurgical instrument. The inventors chose a rotation range of 540 degrees for the instrument tip relative to the flexible shaft. The requirements set by the inventors for the design of an actuator were therefore the following:

- Torque: $56 \times 10^{-3}$ Nm
- Rotation range of instrument tip: 540 degrees
- Maximum outer diameter of actuator: 3.10 mm

The following equations may be used to calculate various properties of the actuator, where the symbols used are summarised in Table 1, below:

$$(1) \quad M = \frac{Ed^4T}{10.8N_tD}$$

$$(2) \quad S = \frac{32M}{\pi d^3}$$

$$(3) \quad M = \frac{Ebt^3T}{6.6N_tD}$$

$$(4) \quad S = \frac{6M}{bt^2}$$

$$(5) \quad E = 83000 \text{ MPa}$$

$$(6) \quad S = 895 \text{ MPa.}$$

Table 1: definition of parameters

| letter | description | units |
|---|---|---|
| D | mean coil diameter | mm |
| d | diameter of round wire with round cross-section | mm |
| $N_t$ | number of coil turns | - |
| E | Young's Modulus | MPa |
| T | deflection, no. of revs of spring | revs |
| S | bending stress | MPa |
| M | moment or torque | N.mm |
| b | Width of wire with rectangular cross-section | mm |
| t | Thickness of wire with rectangular cross-section | mm |

Equations (1) and (2) may be used to describe a helical coil formed of a wire having a circular cross-section, whilst equations (3) and (4) may be used to describe a helical coil formed of a wire having a rectangular cross-section. The values given for (5) and (6) are those for NiTi ("Nitinol") which is used by the inventors for this calculation. The torque requirement for a coil may be halved by using two cooperating helical coils. Thus the torque requirement for one of two cooperating coils is $M = 28 \times 10^{-3}$ Nm.

The outer diameter of a flexible shaft of an electrosurgical instrument may be 2.66 mm. If the coils of the actuator are concentric with the flexible shaft, and the maximum outer diameter is 3.10 mm, the thickness t may be taken to be t = 0.22 mm. The inventors used 75% of the value in (6), as is common practice in spring design. Rearranging equation (4) to find b:

$$(7) \quad b = \frac{6M}{St^2}$$

Using the values mentioned above, one finds b = 5.18 mm. The cross-section of the helical wire is therefore given by t = 0.22 mm and b = 5.18 mm. The next calculation involves determining how many spring turns are needed to accommodate the required rotation of 540 degrees (T = 1.5 revs). The helical wire must fit over the 2.66 mm diameter of the flexible shaft, so D = 2.66 mm + 0.22 mm = 2.88 mm. In the case where the helical wire operates in an unwinding mode (i.e. it unwinds when it is heated up), its diameter will increase as it unwinds. Rearranging equation (3) to find the number of coil turns necessary to accommodate a total deflection of 1.5 revs gives:

$$(8) \quad N_t = \frac{Ebt^3T}{6.6MD}$$

Using the values mentioned above, (8) gives $N_t \approx 13$. The increase in diameter at a full deflection of 1.5 revs may then be calculated. The following formula is used to approximate the inner diameter of the helical coil at full deflection:

$$(9) \quad ID_t = ID * \frac{N_t}{N_t+T}$$

The reciprocal of this equation is used is used in the case where the helical coil unwinds:

$$(10) \quad ID_t = ID * \frac{N_t+T}{N_t}$$

Using the following parameters: ID = 2.66 mm, $N_t$ = 13 mm, and T = 1.5, equation (10) gives $ID_t$ = 2.97 mm, meaning that the outer diameter $OD_t$ of helical coil will be 3.41 mm.
The dimensions calculated above were used to generate a CAD model of the actuator having a cooperating helical structure (like that shown in Fig. 3), in order to calculate the volume of the solid $V = 2.9182 \times 10^{-7}$ m$^{-3}$. The density of Nitinol is 6450 kg/m$^3$. This gives a mass of 1.88 g for the actuator. The specific heat capacity of Nitinol is c = 837.36 J/kg/K. The transition temperature of Nitinol is 40°C, so starting from room temperature a temperature change of $\theta$ = 19°C is required to bring the actuator to the transition temperature. The total energy required to change the temperature of the actuator is given by:

$$(11) \quad H = mass * c * \theta$$

Using equation (11) and the above-mentioned quantities, a total energy of H = 29.91 Joules is required. The CAD model was also used to calculate the length of each of the helical coils (200.2 mm). The resistivity of Nitinol is $\rho_{nitinol} = 9.72 \times 10^{-7}$ Ωm. The electrical resistance of the actuator (taking into account the lengths of both helical coils) is therefore approximately 0.34 Ω. Using the power equation:

$$(12) \quad P = I^2R,$$

a current of I = 9.38 A may be run through the actuator for a period of 1 s to produce the desired temperature increase. Alternatively, the required power may be supplied by higher amplitude current pulses for shorter periods of time, e.g. 29.91 Joules may be delivered as a pulse having a power of 299.1 W for 100 ms.

**Claims**

1. A control mechanism for rotating an instrument tip (204) located at the distal end of an instrument channel in a surgical scoping device, the control mechanism comprising:

   an actuator (216) formed from a shape memory effect material; and
   an energy delivery structure connected to the actuator to deliver energy to cause a change in temperature of the shape memory effect material,
   wherein the actuator is configured to exhibit a torque between a proximal end and a distal end thereof in response to the shape memory effect material reaching a threshold temperature.

2. A control mechanism according to claim 1, wherein the shape memory effect material comprises a helical structure.

3. A control mechanism according to claim 2, wherein the shape memory effect material is configured to unwind upon the temperature of the shape memory effect material reaching the threshold temperature.

4. A control mechanism according to any preceding claim, wherein the shape memory effect material comprises a pair of cooperating helical structures (302, 304).

5. A control mechanism according to any preceding claim including a power source (120) connected to the energy delivery structure.

6. A control mechanism according to any preceding claim, wherein the energy delivery structure comprises at least one of the following:

   (a) a conductive element (224, 226) for running an electrical current through the actuator;
   (b) a heater thermally connected to the actuator.

7. A control mechanism according to any preceding claim including a coolant delivery structure arranged to extract thermal energy from the actuator,

   wherein the coolant delivery structure comprises a coolant circuit in thermal communication with the actuator, and
   wherein the coolant delivery structure is selectively operable.

8. A control mechanism according to any preceding claim wherein the shape memory effect material is at least one of the following:

   (a) a shape memory alloy;
   (b) nitinol.

9. A control mechanism according to any preceding claim, wherein the shape memory effect material exhibits a two-way memory effect to provide bi-directional control of rotation.

10. An electrosurgical instrument (200) for applying radiofrequency (RF) electromagnetic (EM) energy and/or microwave EM energy to biological tissue, the instrument comprising:

    a coaxial cable (208) for conveying the RF EM energy and/or the microwave EM energy;
    an instrument tip (204) connected at a distal end of the coaxial cable to receive the RF EM energy and/or the microwave EM energy and deliver it into biological tissue at a treatment site;
    an elongate shaft (202) defining a lumen (205) for conveying the coaxial cable, wherein the instrument tip protrudes from a distal end of the flexible shaft; and
    a control mechanism according to any one of claims 1 to 9, wherein the proximal end of the actuator is attached to the shaft and the distal end of the actuator is attached to the instrument tip.

11. An electrosurgical instrument according to claim 10, wherein at least one of the following applies:

    (a) the shape memory effect material forms a sleeve around a distal portion of the shaft,
    (b) the shape memory effect material comprises a helical structure wound around an outer surface of the shaft.

12. An electrosurgical instrument according to claim 10 or 11 comprising engagement features (502) at the connection between the shaft and proximal end of the actuator configured to resist relative rotation between the shaft and actuator, and
wherein the engagement features comprise cooperating interengageable elements on the shaft and actuator.

13. An electrosurgical instrument according to any one of claims 10 to 12 comprising engagement features at the connection between the instrument tip and distal end of the actuator configured to resist relative rotation between the instrument tip and actuator, and
wherein the engagement features comprise cooperating interengageable elements on the shaft and actuator.

14. An electrosurgical instrument according to any one of claims 10 to 13 wherein the proximal end of the actuator is attached to the shaft by a weld.

15. An electrosurgical system (100) comprising:

a generator (102) for generating radiofrequency (RF) electromagnetic (EM) energy and/or microwave EM energy;
an electrosurgical instrument according to any one of claims 10 to 14 connected to the generator; and
a surgical scoping device (114) having a manoeuvrable instrument cord with a instrument channel extending therethrough,
wherein the electrosurgical instrument is dimensioned to pass through the instrument channel.


**Patentansprüche**

1. Steuermechanismus zum Rotieren einer Instrumentenspitze (204), die an einem distalen Ende eines Instrumentenkanals in einer chirurgischen Sondierungsvorrichtung angeordnet ist, wobei der Steuermechanismus Folgendes umfasst:

einen Aktuator (216), der aus einem Formgedächtnismaterial ausgebildet ist; und
eine Energiezufuhrstruktur, die mit dem Aktuator verbunden ist, um Energie zuzuführen, um eine Änderung der Temperatur des Formgedächtnismaterials zu bewirken,
wobei der Aktuator ausgelegt ist, um ein Drehmoment zwischen einem proximalen Ende und einem distalen Ende desselben als Reaktion darauf auszubilden, dass das Formgedächtnismaterial eine Schwellentemperatur erreicht.

2. Steuermechanismus nach Anspruch 1, wobei das Formgedächtnismaterial eine Helixstruktur umfasst.

3. Steuermechanismus nach Anspruch 2, wobei das Formgedächtnismaterial ausgelegt ist, um sich zu auseinanderzuwickeln, wenn die Temperatur des Formgedächtnismaterials die Schwellentemperatur erreicht.

4. Steuermechanismus nach einem der vorangegangenen Ansprüche, wobei das Formgedächtnismaterial ein Paar zusammenwirkender Helixstrukturen (302, 304) umfasst.

5. Steuermechanismus nach einem der vorangegangenen Ansprüche, der eine Leistungsquelle (120) aufweist, die mit der Energiezufuhrstruktur verbunden ist.

6. Steuermechanismus nach einem der vorangegangenen Ansprüche, wobei die Energiezufuhrstruktur zumindest eines der folgenden umfasst:

(a) ein leitfähiges Element (224, 226) zum Leiten eines elektrischen Stroms durch den Aktuator;
(b) ein Heizelement, das thermisch mit dem Aktuator verbunden ist.

7. Steuermechanismus nach einem der vorangegangenen Ansprüche, der eine Kühlmittelzufuhrstruktur aufweist, die angeordnet ist, um Wärmeenergie von dem Aktuator abzuführen,

wobei die Kühlmittelzufuhrstruktur einen Kühlmittelkreislauf in thermischer Kommunikation mit dem Aktuator umfasst und
wobei die Kühlmittelzufuhrstruktur selektiv betätigbar ist.

8. Steuermechanismus nach einem der vorangegangenen Ansprüche, wobei das Formgedächtnismaterial zumindest eines der folgenden ist:

    (a) eine Formgedächtnis-Legierung;
    (b) Nitinol.

9. Steuermechanismus nach einem der vorangegangenen Ansprüche, wobei das Formgedächtnismaterial einen Formgedächtnis-Zweiweg-Effekt aufweist, um eine bidirektionale Steuerung der Rotation bereitzustellen.

10. Elektrochirurgisches Instrument (200) zum Anwenden von elektromagnetischer (EM) Hochfrequenz- (HF-) Energie und/oder EM-Mikrowellenenergie auf biologisches Gewebe, wobei das Instrument Folgendes umfasst:

    ein Koaxialkabel (208) zum Übertragen der EM-HF-Energie und/oder der EM-Mikrowellenenergie;
    eine Instrumentenspitze (204), die an einem distalen Ende des Koaxialkabels verbunden ist, um die EM-HF-Energie und/oder die EM-Mikrowellenenergie zu empfangen und an einer Behandlungsstelle in das biologische Gewebe zu leiten;
    einen länglichen Schaft (202), der ein Lumen (205) zum Führen des Koaxialkabels definiert, wobei die Instrumentenspitze aus einem distalen Ende des flexiblen Schafts hervorsteht; und
    einen Steuermechanismus nach einem der Ansprüche 1 bis 9, wobei das proximale Ende des Aktuators an dem Schaft angebracht ist und das distale Ende des Aktuators an der Instrumentenspitze angebracht ist.

11. Elektrochirurgisches Instrument nach Anspruch 10, wobei zumindest eines der folgenden zutrifft:

    (a) das Formgedächtnismaterial bildet eine Hülse rund um einen distalen Abschnitt des Schafts,
    (b) das Formgedächtnismaterial umfasst eine Helixstruktur, die rund um eine Außenfläche des Schafts gewickelt ist.

12. Elektrochirurgisches Instrument nach Anspruch 10 oder 11, umfassend Eingriffselemente (502) an der Verbindung zwischen dem Schaft und dem proximalen Ende des Aktuators, die ausgelegt sind, um einer relativen Rotation zwischen dem Schaft und dem Aktuator zu widerstehen und
    wobei die Eingriffselemente zusammenwirkende, miteinander in Eingriff bringbare Elemente auf dem Schaft und dem Aktuator umfassen.

13. Elektrochirurgisches Element nach einem der Ansprüche 10 bis 12, umfassend Eingriffselemente an der Verbindung zwischen der Instrumentenspitze und dem distalen Ende des Aktuators, die ausgelegt sind, um einer relativen Rotation zwischen der Instrumentenspitze und dem Aktuator zu widerstehen und
    wobei die Eingriffselemente zusammenwirkende miteinander in Eingriff bringbare Elemente auf dem Schaft und dem Aktuator umfassen.

14. Elektrochirurgisches Element nach einem der Ansprüche 10 bis 13, wobei das proximale Ende des Aktuators durch eine Schweißnaht an dem Schaft befestigt ist.

15. Elektrochirurgisches System (100), umfassend:

    einen Generator (102) zum Erzeugen von elektromagnetischer (EM) Hochfrequenz-(HF-) Energie und/oder EM-Mikrowellenenergie;
    ein elektrochirurgisches Instrument nach einem der Ansprüche 10 bis 14, das mit dem Generator verbunden ist; und
    eine chirurgische Sondierungsvorrichtung (114), die ein manövrierbares Instrumentenkabel mit einem Instrumentenkanal aufweist, der sich durch dieses erstreckt,
    wobei das elektrochirurgische Instrument so dimensioniert ist, dass es durch den Instrumentenkanal geführt werden kann.

**Revendications**

1. Mécanisme de commande destiné à faire tourner une pointe d'instrument (204) située au niveau de l'extrémité distale d'un canal d'instrument dans un dispositif d'endoscopie chirurgicale, le mécanisme de commande comprenant :

> un actionneur (216) formé à partir d'un matériau à effet de mémoire de forme ; et
> une structure de fourniture d'énergie connectée à l'actionneur pour fournir de l'énergie pour amener un changement de température du matériau à effet de mémoire de forme,
> dans lequel l'actionneur est configuré pour présenter un couple entre une extrémité proximale et une extrémité distale de celui-ci en réponse au fait que le matériau à effet de mémoire de forme atteint une température seuil.

2. Mécanisme de commande selon la revendication 1, dans lequel le matériau à effet de mémoire de forme comprend une structure hélicoïdale.

3. Mécanisme de commande selon la revendication 2, dans lequel le matériau à effet de mémoire de forme est configuré pour se dérouler lorsque la température du matériau à effet de mémoire de forme atteint la température seuil.

4. Mécanisme de commande selon l'une quelconque des revendications précédentes, dans lequel le matériau à effet de mémoire de forme comprend une paire de structures hélicoïdales coopérantes (302, 304).

5. Mécanisme de commande selon l'une quelconque des revendications précédentes incluant une source d'alimentation (120) connectée à la structure de fourniture d'énergie.

6. Mécanisme de commande selon l'une quelconque des revendications précédentes, dans lequel la structure de fourniture d'énergie comprend au moins l'un des éléments suivants :

> (a) un élément conducteur (224, 226) destiné à faire circuler un courant électrique à travers l'actionneur ;
> (b) un élément chauffant connecté thermiquement à l'actionneur.

7. Mécanisme de commande selon l'une quelconque des revendications précédentes incluant une structure de fourniture de réfrigérant conçue pour extraire de l'énergie thermique de l'actionneur,

> dans lequel la structure de fourniture de réfrigérant comprend un circuit de réfrigérant en communication thermique avec l'actionneur, et
> dans lequel la structure de fourniture de réfrigérant est utilisable de manière sélective.

8. Mécanisme de commande selon l'une quelconque des revendications précédentes dans lequel le matériau à effet de mémoire de forme est au moins l'un des éléments suivants :

> (a) un alliage à mémoire de forme ;
> (b) du nitinol.

9. Mécanisme de commande selon l'une quelconque des revendications précédentes, dans lequel le matériau à effet de mémoire de forme présente un effet de mémoire à double sens pour permettre une commande de rotation bidirectionnelle.

10. Instrument électrochirurgical (200) destiné à appliquer de l'énergie électromagnétique (EM) radiofréquence (RF) et/ou de l'énergie EM micro-onde à un tissu biologique, l'instrument comprenant :

> un câble coaxial (208) destiné à acheminer l'énergie EM RF et/ou l'énergie EM micro-onde ;
> une pointe d'instrument (204) connectée au niveau d'une extrémité distale du câble coaxial pour recevoir l'énergie EM RF et/ou l'énergie EM micro-onde et la fournir au tissu biologique au niveau d'un site de traitement ;
> une tige allongée (202) définissant une lumière (205) destinée à acheminer le câble coaxial, dans lequel la pointe d'instrument fait saillie à partir d'une extrémité distale de la tige flexible ; et
> un mécanisme de commande selon l'une quelconque des revendications 1 à 9, dans lequel l'extrémité proximale de l'actionneur est fixée à la tige et l'extrémité distale de l'actionneur est fixée à la pointe d'instrument.

11. Instrument électrochirurgical selon la revendication 10, dans lequel au moins l'un des points suivants s'applique :

> (a) le matériau à effet de mémoire de forme forme un manchon autour d'une partie distale de la tige,
> (b) le matériau à effet de mémoire de forme comprend une structure hélicoïdale enroulée autour d'une surface externe de la tige.

**12.** Instrument électrochirurgical selon la revendication 10 ou 11 comprenant des accessoires de mise en prise (502) au niveau de la connexion entre la tige et l'extrémité proximale de l'actionneur configurés pour résister à une rotation relative entre la tige et l'actionneur, et
dans lequel les accessoires de mise en prise comprennent des éléments emboîtables coopérants sur la tige et l'actionneur.

**13.** Instrument électrochirurgical selon l'une quelconque des revendications 10 à 12 comprenant des accessoires de mise en prise au niveau de la connexion entre la pointe d'instrument et l'extrémité distale de l'actionneur configurés pour résister à une rotation relative entre la pointe d'instrument et l'actionneur, et
dans lequel les accessoires de mise en prise comprennent des éléments emboîtables coopérants sur la tige et l'actionneur.

**14.** Instrument électrochirurgical selon l'une quelconque des revendications 10 à 13 dans lequel l'extrémité proximale de l'actionneur est fixée à la tige par une soudure.

**15.** Système électrochirurgical (100) comprenant :

un générateur (102) destiné à générer de l'énergie électromagnétique (EM) radiofréquence (RF) et/ou de l'énergie EM micro-onde ;
un instrument électrochirurgical selon l'une quelconque des revendications 10 à 14 connecté au générateur ; et
un dispositif d'endoscopie chirurgicale (114) ayant un cordon d'instrument manoeuvrable avec un canal d'instrument s'étendant à travers lui,
dans lequel l'instrument électrochirurgical est dimensionné pour passer à travers le canal d'instrument.

FIG. 1

FIG. 2

FIG. 3

306 — 304

312 —

402

302

FIG. 4A

309

310

302 —

304 —

404    308

FIG. 4B

500

502

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016059228 A
- US 4790624 A
- WO 2012076844 A
- WO 2014006369 A